# EUROPEAN PATENT APPLICATION

(11) **EP 4 434 620 A1**
(43) Date of publication of application: **25.09.2024**
(21) Application number: 22914321.9
(22) Date of filing: 16.12.2022
(51) Int. Cl.: B01J 23/75, B01J 23/72, C07D 307/68

(54) **CATALYST, PREPARATION METHOD THEREFOR AND METHOD FOR PREPARING 2,5-FURANDICARBOXYLIC ACID FROM CATALYST**

(30) Priority: 30.12.2021 CN 202111660696
(71) Applicant: PetroChina Company Limited, Dongcheng District Beijing 100007 (CN)
(72) Inventor: JIANG, Wei, Beijing 100724 (CN); XIN, Ying, Beijing 100724 (CN); JIN, Shuhan, Beijing 100724 (CN); GONG, Mingyue, Beijing 100724 (CN); WANG, Weizhong, Beijing 100724 (CN); WANG, Dongjun, Beijing 100724 (CN); LI, Yinghui, Beijing 100724 (CN); ZHAO, Chenkang, Beijing 100724 (CN); DENG, Xuliang, Beijing 100724 (CN); MENG, Rui, Beijing 100724 (CN)
(74) Representative: Metroconsult Srl
(86) International application number: PCT/CN2022/139528
(87) International publication number: WO 2023/125067

(57) **Abstract**

A CoCuₓO_{y} catalyst, the molar ratio of Co, Cu and O thereof being 1:(0.2-1):(3-4). A preparation method of the catalyst comprises: dropwise adding a solution of an oxidizing agent and an initiator into a mixed aqueous solution of copper nitrate and cobalt nitrate, filtering the mixed solution after ageing to obtain a black cake-like precipitate, and calcining the precipitate at 180°C to obtain the CoCuₓO_{y} catalyst. The present invention also provides a method for preparing 2,5-furandicarboxylic acid from the CoCuₓO_{y} catalyst prepared by said method. The preparation method of the CoCuₓO_{y} catalyst in the present invention has simple and convenient operations and mild conditions; the prepared catalyst has high catalytic efficiency; and using the catalyst for preparing 2,5-furandicarboxylic acid is characterized by high efficiency, easy separation, low costs, less pollution and the like.

## Description

### Field of Technology

The present disclosure belongs to the field of biomass energy, and specifically relates to a CoCuₓO_{y} catalyst and its preparation method and a method for the catalytic preparation of 2,5-furandicarboxylic acid using the catalyst.

### Background Art

Biomass, as the only renewable carbon resource, can be converted into high value-added fuels and chemicals, which can play an important role in solving energy and environmental problems. 2,5-furandicarboxylic acid (FDCA) is one of the potential bio-based platform compounds. The current preparation methods of FDCA are mainly classified as follows: a preparation of furoic acid by disproportionation reaction or chloromethylation reaction or CO₂ solid-phase reaction; a preparation of hexanoic acid by oxidative dehydration reaction; a preparation by reaction of diethanolic acid being a raw material with methanol and thionyl chloride to produce dimethyl diethanolate, which is then subjected to dehydration and condensation with trimeric glyoxal; a preparation by using 2,5-dimethylfuran, 2-methyl-5-acetylfuran or 5-methyl-2-fural as a raw material; a preparation by oxidation of 5-hydroxymethylfurfural (HMF) obtained by hydrolysis of hexose. The structure of HMF has similar functional group positions with FDCA, and thus the preparation of FDCA by HMF is currently the most studied and optimal choice of raw material, and it is a more promising route for the preparation of FDCA.

At present, 2,5-furandicarboxylic acid is mainly prepared from the oxidation of 5-hydroxymethylfurfural (HMF), which generally requires the addition of catalysts, which are divided into heterogeneous and homogeneous catalysts. Homogeneous catalysts are mainly salts of transition metal such as Co and Mn. For example, Grushin's group used cobalt salt homogeneous catalytic oxidation of HMF, in which the initial concentration of HMF was about 8%, and the Co/Mn/Br/Zr catalytic reaction was carried out for 3 hours at an air of 7 MPa and 125°C, and the yield of FDCA obtained was 60% (W. Partenheimer, V.V.Grushin,Adv.Synth.Catal.,2001,343:102-111). The homogeneous catalytic reaction system has the disadvantages of low yield, difficult separation of metal salts, and serious pollution of bromine environment. In contrast, the heterogeneous catalytic method has the advantages of high selectivity, environmental protection, and easy separation of products. For the heterogeneous catalytic oxidation of HMF, two main types of precious metal catalysts and non-precious metal catalysts are mainly used. The noble metal catalysts include Pt, Au, Pd and the like. For example, Xuewang Han et al. used carbon-magnesium oxide as an alkaline carrier to support the noble metal Pt, in which the FDCA yield could reach 95% under alkali-free conditions (Xuewang Han, Liang Geng, Green Chemistry, 2015, 14:4-17). Lin lu et al. in their patent (CN 201010228459) prepared FDCA by catalytic oxidation of HMF in alkaline solution using Pt, Au, and Pd-supported C or CuO-Ag₂O as catalysts to obtain high FDCA yields. Existing non-precious metal heterogeneous catalysts generally suffer from low selectivity and poor stability under oxygen or air conditions. For example, Zehui Zhang 's group catalyzed the oxidation of HMF with nano-Fe₃O₄-CoOₓ for the preparation of FDCA, in which the FDCA yield could reach 68.6% when tert-butanol peroxide was used as the oxidizing agent, but the FDCA yield was only 4.2% when oxygen was used as the oxidizing agent (Shuguo Wang, Zehui Zhang, ACS Sustainable Chem. Eng. 2015,3:406-412).

### Summary

In order to solve the above problems, it is an object of the present disclosure to provide a CoCuₓO_{y} catalyst and its preparation method, and a preparation method of 2,5-furandicarboxylic acid by using such catalyst. The preparation of 2,5-furandicarboxylic acid by using such catalyst is characterized by high efficiency, easy separation, low cost, low pollution, and the like.

In order to achieve the above object, the present disclosure provides a CoCuₓO_{y} catalyst, wherein the molar ratio of Co:Cu:O in the catalyst is 1:(0.2-1) : (3-4).

The present disclosure further provides a preparation method of the CoCuₓO_{y} catalyst, which comprises the following steps:
a soluble cobalt salt and a soluble copper salt are dispersed in water at a mass ratio of 1:1-2, in which the total mass concentration of these two salts is controlled to be less than 10%; subsequently, an aqueous solution of a first oxidizing agent and an initiator at a certain mass concentration is weighed and added dropwise to the mixed aqueous solution of the soluble cobalt salt and the soluble copper salt at a flow rate of 2-5 drops/second; after dropwise addition, the mixture is continued to be aged for 2-6 hours (preferably 4-6 hours) and filtered to produce a black cake-like precipitate, which was roasted in an air atmosphere to obtain the CoCuₓO_{y} catalyst.

In the preparation method, preferably, the first oxidizing agent is one or more of perchloric acid, potassium perchlorate, sodium perchlorate, ammonium perchlorate, calcium perchlorate, and magnesium perchlorate.

In the preparation method, preferably, the initiator is one or more of N,N-diethylacrylamide, N,N-dimethylacrylamide, and N-isopropylacrylamide.

In the preparation method, preferably, the soluble cobalt salt is one or more of cobalt chloride, cobalt bromide, cobalt nitrate, and cobalt sulfate.

In the preparation method, preferably, the soluble copper salt is one or more of copper chloride, copper bromide, copper sulfate, and copper nitrate.

In the preparation method, preferably, the mass ratio of the first oxidizing agent and the initiator is 1:1.

In the preparation method, preferably, the mass concentration of the first oxidizing agent and the initiator is 5-30%.

In the preparation method, preferably, the mass concentration of the first oxidizing agent and the initiator is 10-20%.

According to a specific embodiment of the present disclosure, preferably, the preparation method includes the following steps:
cobalt nitrate and copper nitrate are dispersed in deionized water at a mass ratio of 1:1-2, in which the total mass concentration of these two salts is controlled to be less than 10%; subsequently, a solution of calcium perchlorate and N,N-diethylacrylamide at a mass concentration of 10% was weighed and added dropwise to the mixed aqueous solution of copper nitrate and cobalt nitrate with a mass concentration of 10% at a flow rate of 2-5 drops/second; after dropwise addition, the mixture is continued to be aged for 4-6 hours and filtered to produce a black cake-like precipitate, which is roasted in an air atmosphere to obtain the CoCuₓO_{y} catalyst.

In the preparation method, preferably, the mass ratio of calcium perchlorate and N,N-diethylacrylamide is 1:1.

In the preparation method, preferably, the solution of calcium perchlorate and N,N-diethylacrylamide is used at an amount of 20 mL.

In the preparation method, preferably, the roasting is carried out at a temperature of 180°C for 1.5-2.5 hours.

In the preparation method, preferably, the mass concentration of calcium perchlorate and N,N-diethylacrylamide is 5-30%.

In the preparation method, preferably, the mass concentration of calcium perchlorate and N,N-diethylacrylamide is 10-20%.

The present disclosure further provides a preparation method of 2,5-furandicarboxylic acid through catalytic oxidation, in which the catalytic reaction is carried out with the CoCuₓO_{y} catalyst of the present disclosure, the method comprises the following steps:
5-hydroxymethylfurfural, which serves as a raw material, is mixed with water, the CoCuₓO_{y} catalyst and a second oxidizing agent at a certain ratio in a reactor, allowed to react at 20-90°C for 10-600 min, and then cooled to room temperature to obtain 2,5-furandicarboxylic acid reaction solution; the reaction solution is filtered and then the pH is adjusted to 1-2 (preferably 1) by adding an appropriate amount of acid to obtain precipitates, which are centrifuged or filtered, followed by suction filtration and vacuum drying, to obtain 2,5-furandicarboxylic acid.

In the preparation method, preferably, the second oxidizing agent is one or more of NaClO, KClO and ClO₂.

In the preparation method, preferably, a mass concentration (relative to the total mass of the reaction system) of the 5-hydroxymethylfurfural is 0.5-25%, preferably 10-20%.

In the preparation method, preferably, a mass ratio of the catalyst to 5-hydroxymethylfurfural is 1:1.

In the preparation method, preferably, a molar ratio of the second oxidizing agent and 5-hydroxymethylfurfural is (1-20): 1, preferably (3-10): 1.

In the preparation method, preferably, the acid used to adjust the pH is one or more of hydrochloric acid, sulfuric acid, and phosphoric acid.

In the preparation method, preferably, the acid used to adjust the pH is in a form of an aqueous solution with a mass concentration of 50%.

In the preparation method, preferably, the reaction temperature is 25-35°C.

In the preparation method, preferably, the reaction duration is 20-180 minutes.

According to a specific embodiment of the present disclosure, the preparation method may be carried out in accordance with the following specific steps:
the biomass platform compound 5-hydroxymethylfurfural, which serves as a raw material, is mixed with water, the CoCuₓO_{y} catalyst and the second oxidizing agent at a certain ratio in a reactor, allowed to react at 25-70°C for 60-300 min, and then cooled to room temperature to obtain 2,5-furandicarboxylic acid reaction solution; the reaction solution is filtered and then the pH thereof is adjusted to 1-2 by adding an appropriate amount of acid, to obtain precipitates, which are centrifuged or filtered to obtain 2,5-furandicarboxylic acid; the raw 2,5-furandicarboxylic acid is finally rinsed with deionized water, suction filtered, and vacuum dried to obtain a milky white solid powder of 2,5-furandicarboxylic acid.

According to a specific embodiment of the present disclosure, preferably, the preparation method may be carried out in accordance with the following specific steps:
the biomass platform compound 5-hydroxymethylfurfural, which serves as a raw material, is mixed with water, the CoCuₓO_{y} catalyst and the second oxidizing agent at a certain ratio in a reactor, allowed to react at 30°C for 180 min, and then cooled to room temperature to obtain 2,5-furandicarboxylic acid reaction solution; the reaction solution is filtered and then the pH thereof is adjusted to 1 by adding appropriate amount of acid to obtain precipitates, which are centrifuged or filtered to obtain 2,5-furandicarboxylic acid; The raw 2,5-furandicarboxylic acid is finally rinsed with deionized water, suction filtered and vacuum dried to obtain a milky white solid powder of 2,5-furandicarboxylic acid.

The catalytic preparation of 2,5-furandicarboxylic acid by the catalyst of the present disclosure has the following beneficial effects:
The catalyst is used at a small amount, and the catalytic reaction system is simple and efficient, with mild reaction conditions and few by-products. According to chromatographic analysis, the conversion rate was more than 99%. The purity of the product after separation and purification was up to more than 99.5%.

### Brief Description of The Drawings

Figure 1 is a ¹H-NMR (DMSO-d₆) NMR plot of 2,5-furandicarboxylic acid, the separated product in Example 17 of the present disclosure.
Figure 2 is a ¹³C-NMR (DMSO-d₆) NMR plot of 2,5-furandicarboxylic acid, the separated product in Example 17 of the present disclosure.

### Detailed Description of Preferred Embodiments

In order to enable a person skilled in the art to better understand the technical solutions in the present application, the present disclosure will be described more clearly and completely hereinafter in conjunction with specific examples of the present disclosure. Obviously, the described examples are only a part of, not all of the examples of the present disclosure. Based on the examples in the present disclosure, all other embodiments obtained by an ordinary person skilled in the art without making creative labor should fall within the protection scope of the present disclosure.

### Preparation Examples of the CoCuₓO_{y} catalyst

### Example 1

Cobalt nitrate and copper nitrate were dispersed in deionized water, wherein the mass concentration of cobalt nitrate was 5% and the mass concentration of copper nitrate was 5%. Subsequently, 20 ml of a solution of calcium perchlorate and N,N-diethylacrylamide with a mass concentration of 10% was weighted and added dropwise to the mixed aqueous solution of copper nitrate and cobalt nitrate at a flow rate of 3 drops/second. After dropwise addition, the mixture was then aged for 4 hours and filtered to produce a black cake-like precipitate, which was then roasted in an air atmosphere at 180°C for 2 hours, to obtain the CoCuₓO_{y} catalyst CAT-1.

The molar ratio of Co:Cu:O in the catalyst was characterized by analysis as 1:0.8:3.5.

### Example 2

Cobalt nitrate and copper nitrate were dispersed in deionized water, wherein the mass concentration of cobalt nitrate was 3% and the mass concentration of copper nitrate was 6%. Subsequently, 20 ml of a solution of calcium perchlorate and N,N-diethylacrylamide with a mass concentration of 10% was weighted and added dropwise to the mixed aqueous solution of copper nitrate and cobalt nitrate at a flow rate of 2 drops/second. After dropwise addition, the mixture was then aged for 4 hours and filtered to produce a black cake-like precipitate, which was then roasted in an air atmosphere at 180°C for 2 hours, to obtain the CoCuₓO_{y} catalyst CAT-2.

The molar ratio of Co:Cu:O in the catalyst was characterized by analysis as 1:0.9:3.9.

### Example 3

Cobalt chloride and copper chloride were dispersed in deionized water, wherein the mass concentration of cobalt chloride was 4% and the mass concentration of copper chloride was 5%. Subsequently, 20 ml of a solution of potassium perchlorate and N,N-diethylacrylamide with a mass concentration of 10% was weighted and added dropwise to the mixed aqueous solution of cobalt chloride and copper chloride at a flow rate of 3 drops/second. After dropwise addition, the mixture was then aged for 4 hours and filtered to produce a black cake-like precipitate, which was then roasted in an air atmosphere at 200°C for 2 hours, to obtain the CoCuₓO_{y} catalyst CAT-3.

The molar ratio of Co:Cu:O in the catalyst was characterized by analysis as 1:0.6: 3.1.

### Example 4

Cobalt chloride and copper chloride were dispersed in deionized water, wherein the mass concentration of cobalt chloride was 6% and the mass concentration of copper chloride was 3%. Subsequently, 20 ml of a solution of calcium perchlorate and N-isopropylacrylamide with a mass concentration of 15% was weighted and added dropwise to the mixed aqueous solution of cobalt chloride and copper chloride at a flow rate of 4 drops/second. After dropwise addition, the mixture was then aged for 4 hours and filtered to produce a black cake-like precipitate, which was then roasted in an air atmosphere at 180°C for 2 hours, to obtain the CoCuₓO_{y} catalyst CAT-4.

The molar ratio of Co:Cu:O in the catalyst was characterized by analysis as 1:0.7:2.9.

### Example 5

Cobalt sulfate and copper sulfate were dispersed in deionized water, wherein the mass concentration of cobalt sulfate was 2% and the mass concentration of copper sulfate was 5%. Subsequently, 20 ml of a solution of magnesium perchlorate and N,N-dimethylacrylamide with a mass concentration of 10% was weighted and added dropwise to the mixed aqueous solution of cobalt sulfate and copper sulfate at a flow rate of 4 drops/second. After dropwise addition, the mixture was then aged for 3 hours and filtered to produce a black cake-like precipitate, which was then roasted in an air atmosphere at 180°C for 2.5 hours, to obtain the CoCuₓO_{y} catalyst CAT-5.

The molar ratio of Co:Cu:O in the catalyst was characterized by analysis as 1:0.5:2.7.

### Example 6

Cobalt nitrate and copper sulfate were dispersed in deionized water, wherein the mass concentration of cobalt nitrate was 6% and the mass concentration of copper sulfate was 5%. Subsequently, 20 ml of a solution of magnesium perchlorate and N,N-diethylacrylamide with a mass concentration of 15% was weighted and added dropwise to the mixed aqueous solution of cobalt nitrate and copper sulfate at a flow rate of 3 drops/second. After dropwise addition, the mixture was then aged for 2 hours and filtered to produce a black cake-like precipitate, which was then roasted in an air atmosphere at 180°C for 2.5 hours, to obtain the CoCuₓO_{y} catalyst CAT-6.

The molar ratio of Co:Cu:O in the catalyst was characterized by analysis as 1:0.8:3.2.

### Example 7

Cobalt bromide and copper sulfate were dispersed in deionized water, wherein the mass concentration of cobalt bromide was 5% and the mass concentration of copper sulfate was 5%. Subsequently, 20 ml of a solution of calcium perchlorate and N,N-diethylacrylamide with a mass concentration of 25% was weighted and added dropwise to the mixed aqueous solution of cobalt bromide and copper sulfate at a flow rate of 4 drops/second. After dropwise addition, the mixture was then aged for 2 hours and filtered to produce a black cake-like precipitate, which was then roasted in an air atmosphere at 200°C for 2 hours, to obtain the CoCuₓO_{y} catalyst CAT-7.

The molar ratio of Co:Cu:O in the catalyst was characterized by analysis as 1:0.7:3.0.

### Example 8

Cobalt bromide and copper chloride were dispersed in deionized water, wherein the mass concentration of cobalt bromide was 5% and the mass concentration of copper chloride was 3%. Subsequently, 20 ml of a solution of sodium perchlorate and N,N-diethylacrylamide with a mass concentration of 25% was weighted and added dropwise to the mixed aqueous solution of cobalt bromide and copper chloride at a flow rate of 4 drops/second. After dropwise addition, the mixture was then aged for 2 hours and filtered to produce a black cake-like precipitate, which was then roasted in an air atmosphere at 200°C for 3 hours, to obtain the CoCuₓO_{y} catalyst CAT-8.

The molar ratio of Co:Cu:O in the catalyst was characterized by analysis as 1:0.9:3.3.

### Example 9

Cobalt nitrate and copper chloride were dispersed in deionized water, wherein the mass concentration of cobalt bromide was 3% and the mass concentration of copper chloride was 7%. Subsequently, 15 ml of a solution of calcium perchlorate and N-isopropylacrylamide with a mass concentration of 20% was weighted and added dropwise to the mixed aqueous solution of cobalt nitrate and copper chloride at a flow rate of 4 drops/second. After dropwise addition, the mixture was then aged for 2 hours and filtered to produce a black cake-like precipitate, which was then roasted in an air atmosphere at 200°C for 2 hours, to obtain the CoCuₓO_{y} catalyst CAT-9.

The molar ratio of Co:Cu:O in the catalyst was characterized by analysis as 1:0.7:2.4.

### Example 10

Cobalt bromide and copper nitrate were dispersed in deionized water, wherein the mass concentration of cobalt bromide was 5% and the mass concentration of copper nitrate was 7%. Subsequently, 20 ml of a solution of calcium perchlorate and N-isopropylacrylamide with a mass concentration of 20% was weighted and added dropwise to the mixed aqueous solution of cobalt bromide and copper nitrate at a flow rate of 3 drops/second. After dropwise addition, the mixture was then aged for 2 hours and filtered to produce a black cake-like precipitate, which was then roasted in an air atmosphere at 180°C for 2 hours, to obtain the CoCuₓO_{y} catalyst CAT-10.

The molar ratio of Co:Cu:O in the catalyst was characterized by analysis as 1:0.9:3.5.

### Example 11

Copper nitrate and cobalt nitrate were dispersed in deionized water, wherein the mass concentration of cobalt nitrate was 3% and the mass concentration of copper nitrate was 6%. Subsequently, 20 ml of a solution of calcium perchlorate and N,N-diethylacrylamide with a mass concentration of 10% was weighted and added dropwise to the mixed aqueous solution of copper nitrate and cobalt nitrate at a flow rate of 4 drops/second. After dropwise addition, the mixture was then aged for 6 hours and filtered to produce a black cake-like precipitate, which was then roasted in an air atmosphere at 180°C for 1.5 hours, to obtain the CoCuₓO_{y} catalyst.

The molar ratio of Co:Cu:O in the catalyst was characterized by analysis as 1:0.7:3.2.

### Example 12

Cobalt nitrate and copper nitrate were dispersed in deionized water, wherein the mass concentration of cobalt nitrate was 3% and the mass concentration of copper nitrate was 4%. Subsequently, 20 ml of a solution of calcium perchlorate and N,N-diethylacrylamide with a mass concentration of 10% was weighted and added dropwise to the mixed aqueous solution of cobalt nitrate and copper nitrate at a flow rate of 4 drops/second. After dropwise addition, the mixture was then aged for 5 hours and filtered to produce a black cake-like precipitate, which was then roasted in an air atmosphere at 180°C for 2.5 hours, to obtain the CoCuₓO_{y} catalyst.

The molar ratio of Co:Cu:O in the catalyst was characterized by analysis as 1:0.9:3.1.

### Example 13

Cobalt nitrate and copper nitrate were dispersed in deionized water, wherein the mass concentration of cobalt nitrate was 1% and the mass concentration of copper nitrate was 2%. Subsequently, 20 ml of a solution of calcium perchlorate and N,N-diethylacrylamide with a mass concentration of 10% was weighted and added dropwise to the mixed aqueous solution of cobalt nitrate and copper nitrate at a flow rate of 2 drops/second. After dropwise addition, the mixture was then aged for 4 hours and filtered to produce a black cake-like precipitate, which was then roasted in an air atmosphere at 180°C for 1.5 hours, to obtain the CoCuₓO_{y} catalyst.

The molar ratio of Co:Cu:O in the catalyst was characterized by analysis as 1:0.6:3.0.

### Example 14

Cobalt nitrate and copper nitrate were dispersed in deionized water, wherein the mass concentration of cobalt nitrate was 3% and the mass concentration of copper nitrate was 6%. Subsequently, 20 ml of a solution of calcium perchlorate and N,N-diethylacrylamide with a mass concentration of 10% was weighted and added dropwise to the mixed aqueous solution of cobalt nitrate and copper nitrate at a flow rate of 5 drops/second. After dropwise addition, the mixture was then aged for 6 hours and filtered to produce a black cake-like precipitate, which was then roasted in an air atmosphere at 180°C for 2.5 hours, to obtain the CoCuₓO_{y} catalyst.

The molar ratio of Co:Cu:O in the catalyst was characterized by analysis as 1:0.9:3.4.

### Example 15

Cobalt nitrate and copper nitrate were dispersed in deionized water, wherein the mass concentration of cobalt nitrate was 5% and the mass concentration of copper nitrate was 5%. Subsequently, 20 ml of a solution of calcium perchlorate and N,N-diethylacrylamide with a mass concentration of 10% was weighted and added dropwise to the mixed aqueous solution of cobalt nitrate and copper nitrate at a flow rate of 2 drops/second. After dropwise addition, the mixture was then aged for 6 hours and filtered to produce a black cake-like precipitate, which was then roasted in an air atmosphere at 180°C for 1.5 hours, to obtain the CoCuₓO_{y} catalyst.

The molar ratio of Co:Cu:O in the catalyst was characterized by analysis as 1:0.4:3.0.

### Preparation Examples of 2,5-furandicarboxylic acid

### Example 16

The preparation of 2,5-furandicarboxylic acid provided in this example was carried out by the following steps: 0.25 g of 5-hydroxymethylfurfural, 0.125 g of the catalyst CAT-1 (the molar ratio of Co:Cu:O is 1:0.8:3.5), 50 ml of sodium hypochlorite solution with a mass concentration of 5.7% were added to a 100 ml round-bottom flask and allowed to react at 0°C for 300 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 118.1%.

The mass yield of 2,5-furandicarboxylic acid was calculated according to the following equation: Conversion rate of 5-hydroxymethylfurfular (C_HMF, mol%) = (1 - mass of HMF in the product/initial mass of HMF) × 100% Yield of 2,5-furadicarboxylic acid (Y_X, mol%) = mass of 2,5-furadicarboxylic acid in the product/(initial mass of 5-hydroxymethylfurfural) × 100%

### Example 17

The preparation of 2,5-furandicarboxylic acid provided in this example was carried out by the following steps: 0.5 g of 5-hydroxymethylfurfural, 0.3 g of the catalyst CoCuₓO_{y} (the molar ratio of Co:Cu:O is 1:0.8:3.5), 50 ml of sodium hypochlorite solution with a mass concentration of 5.7% were added to a 100 ml round-bottom flask and allowed to react at 5°C for 270 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 119.2% (the calculation methods are the same as those in Example 16).

### Example 18

The preparation of 2,5-furandicarboxylic acid provided in this example was carried out by the following steps: 0.75 g of 5-hydroxymethylfurfural, 0.525 g of the catalyst CoCuₓO_{y} (the molar ratio of Co:Cu:O is 1:0.7:3.2), 50 ml of sodium hypochlorite solution with a mass concentration of 8.3% were added to a 100 ml round-bottom flask and allowed to react at 10°C for 240 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 114.0% (the calculation methods are the same as those in Example 16).

### Example 19

The preparation of 2,5-furandicarboxylic acid provided in this example was carried out by the following steps: 1.0 g of 5-hydroxymethylfurfural, 0.8 g of the catalyst CoCuₓO_{y} (the molar ratio of Co:Cu:O is 1:0.9:3.9), 50 ml of sodium hypochlorite solution with a mass concentration of 10.6% were added to a 100 ml round-bottom flask and allowed to react at 15°C for 200 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 115.7% (the calculation methods are the same as those in Example 16).

### Example 20

The preparation of 2,5-furandicarboxylic acid provided in this example was carried out by the following steps: 1.25 g of 5-hydroxymethylfurfural, 1.125 g of the catalyst CoCuₓO_{y} (the molar ratio of Co:Cu:O is 1:0.9:3.1), 50 ml of sodium hypochlorite solution with a mass concentration of 12.8% were added to a 100 ml round-bottom flask and allowed to react at 17.5°C for 180 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 116.7% (the calculation methods are the same as those in Example 16).

### Example 21

The preparation of 2,5-furandicarboxylic acid provided in this example was carried out by the following steps: 1.5 g of 5-hydroxymethylfurfural, 1.5 g of the catalyst CoCuₓO_{y} (the molar ratio of Co:Cu:O is 1:0.6:3.0), 50 ml of sodium hypochlorite solution with a mass concentration of 14.8% were added to a 100 ml round-bottom flask and allowed to react at 20°C for 150 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 114.9% (the calculation methods are the same as those in Example 16).

### Example 22

The preparation of 2,5-furandicarboxylic acid provided in this example was carried out by the following steps: 1.75 g of 5-hydroxymethylfurfural, 1.93 g of the catalyst CoCuₓO_{y} (the molar ratio of Co:Cu:O is 1:0.9:3.4), 50 ml of sodium hypochlorite solution with a mass concentration of 16.5% were added to a 100 ml round-bottom flask and allowed to react at 22.5°C for 120 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 119.2% (the calculation methods are the same as those in Example 16).

### Example 23

The preparation of 2,5-furandicarboxylic acid provided in this example was carried out by the following steps: 2.0 g of 5-hydroxymethylfurfural, 2.4 g of the catalyst CoCuₓO_{y} (the molar ratio of Co:Cu:O is 1:0.6:3.0), 50 ml of sodium hypochlorite solution with a mass concentration of 18.2% were added to a 100 ml round-bottom flask and allowed to react at 60°C for 10 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 118.6% (the calculation methods are the same as those in Example 16).

### Example 24

The preparation of 2,5-furandicarboxylic acid provided in this example was carried out by the following steps: 2.25 g of 5-hydroxymethylfurfural, 2.8 g of the catalyst CoCuₓO_{y} (the molar ratio of Co:Cu:O is 1:0.7:3.2), 50 ml of sodium hypochlorite solution with a mass concentration of 19.5% were added to a 100 ml round-bottom flask and allowed to react at 57.5°C for 13 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 120.8% (the calculation methods are the same as those in Example 16).

### Example 25

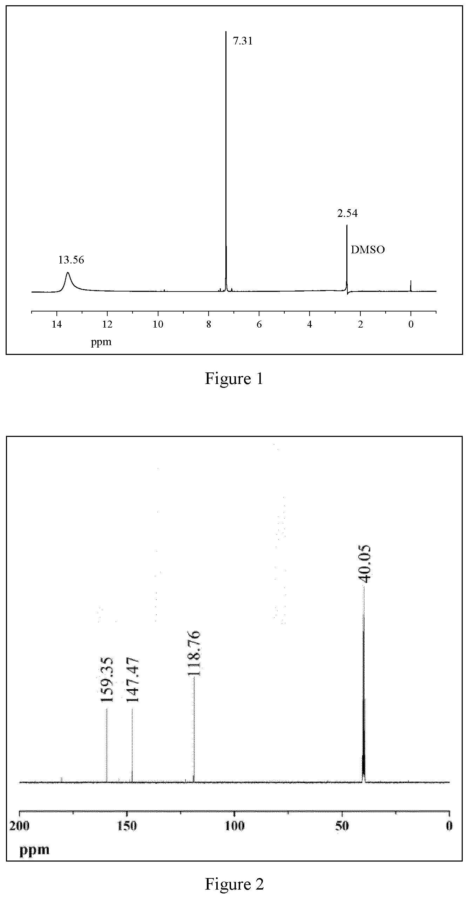

The preparation of 2,5-furandicarboxylic acid provided in this example was carried out by the following steps: 2.5 g of 5-hydroxymethylfurfural, 3.25 g of the catalyst CoCuₓO_{y} (the molar ratio of Co:Cu:O is 1:0.8:3.5), 50 ml of sodium hypochlorite solution with a mass concentration of 20.7% were added to a 100 ml round-bottom flask and allowed to react at 55°C for 15 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 122.4% (the calculation methods are the same as those in Example 16). The ¹H-NMR plot of 2,5-furandicarboxylic acid obtained in this example is shown in Figure 1, and its ¹³C-NMR plot is shown in Figure 2.

### Example 26

The preparation of 2,5-furandicarboxylic acid provided in this example was carried out by the following steps: 2.75 g of 5-hydroxymethylfurfural, 1.35 g of the catalyst CoCuₓO_{y} (the molar ratio of Co:Cu:O is 1:0.9:3.9), 50 ml of sodium hypochlorite solution with a mass concentration of 21.8% were added to a 100 ml round-bottom flask and allowed to react at 52.5°C for 18 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 112.6% (the calculation methods are the same as those in Example 16).

### Example 27

The preparation of 2,5-furandicarboxylic acid provided in this example was carried out by the following steps: 3.0 g of 5-hydroxymethylfurfural, 4.2 g of the catalyst CoCuₓO_{y} (the molar ratio of Co:Cu:O is 1:0.9:3.9), 50 ml of sodium hypochlorite solution with a mass concentration of 22.5% were added to a 100 ml round-bottom flask and allowed to react at 50°C for 20 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 113.9% (the calculation methods are the same as those in Example 16).

### Example 28

The preparation of 2,5-furandicarboxylic acid provided in this example was carried out by the following steps: 3.25 g of 5-hydroxymethylfurfural, 4.7 g of the catalyst CoCuₓO_{y} (the molar ratio of Co:Cu:O is 1:0.9:3.1), 50 ml of sodium hypochlorite solution with a mass concentration of 23.0% were added to a 100 ml round-bottom flask and allowed to react at 47.5°C for 16 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 115.5% (the calculation methods are the same as those in Example 16).

### Example 29

The preparation of 2,5-furandicarboxylic acid provided in this example was carried out by the following steps: 3.5 g of 5-hydroxymethylfurfural, 5.25 g of the catalyst CoCuₓO_{y} (the molar ratio of Co:Cu:O is 1:0.8:3.5), 50 ml of sodium hypochlorite solution with a mass concentration of 23.6% were added to a 100 ml round-bottom flask and allowed to react at 45°C for 14 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 113.0% (the calculation methods are the same as those in Example 16).

### Example 30

The preparation of 2,5-furandicarboxylic acid provided in this example was carried out by the following steps: 3.75 g of 5-hydroxymethylfurfural, 5.8 g of the catalyst CoCuₓO_{y} (the molar ratio of Co:Cu:O is 1:0.8:3.5), 50 ml of sodium hypochlorite solution with a mass concentration of 23.7% were added to a 100 ml round-bottom flask and allowed to react at 40°C for 22 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 111.3% (the calculation methods are the same as those in Example 16).

### Example 31

The preparation of 2,5-furandicarboxylic acid provided in this example was carried out by the following steps: 4.0 g of 5-hydroxymethylfurfural, 6.4 g of the catalyst CoCuₓO_{y} (the molar ratio of Co:Cu:O is 1:0.6:3.0), 50 ml of sodium hypochlorite solution with a mass concentration of 23.6% were added to a 100 ml round-bottom flask and allowed to react at 37.5°C for 25 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 108.2% (the calculation methods are the same as those in Example 16).

### Example 32

The preparation of 2,5-furandicarboxylic acid provided in this example was carried out by the following steps: 4.25 g of 5-hydroxymethylfurfural, 7.0 g of the catalyst CoCuₓO_{y} (the molar ratio of Co:Cu:O is 1:0.6:3.0), 50 ml of sodium hypochlorite solution with a mass concentration of 22.6% were added to a 100 ml round-bottom flask and allowed to react at 35°C for 30 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 107.3% (the calculation methods are the same as those in Example 16).

### Example 33

The preparation of 2,5-furandicarboxylic acid provided in this example was carried out by the following steps: 4.5 g of 5-hydroxymethylfurfural, 7.65 g of the catalyst CoCuₓO_{y} (the molar ratio of Co:Cu:O is 1:0.9:3.9), 50 ml of sodium hypochlorite solution with a mass concentration of 21.25% were added to a 100 ml round-bottom flask and allowed to react at 30°C for 35 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 103.4% (the calculation methods are the same as those in Example 16).

### Example 34

The preparation of 2,5-furandicarboxylic acid provided in this example was carried out by the following steps: 4.75 g of 5-hydroxymethylfurfural, 0.8.55 g of the catalyst CoCuₓO_{y} (the molar ratio of Co:Cu:O is 1:0.9:3.9), 50 ml of sodium hypochlorite solution with a mass concentration of 19.6% were added to a 100 ml round-bottom flask and allowed to react at 27.5°C for 40 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 102.0% (the calculation methods are the same as those in Example 16).

### Example 35

The preparation of 2,5-furandicarboxylic acid provided in this example was carried out by the following steps: 5.0 g of 5-hydroxymethylfurfural, 10.0 g of the catalyst CoCuₓO_{y} (the molar ratio of Co:Cu:O is 1:0.9:3.4), 50 ml of sodium hypochlorite solution with a mass concentration of 17.7% were added to a 100 ml round-bottom flask and allowed to react at 25°C for 45 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 100.0% (the calculation methods are the same as those in Example 16).

### Example 36

The preparation of 2,5-furandicarboxylic acid provided in this example was carried out by the following steps: 5.6 g of 5-hydroxymethylfurfural, 1.4 g of the catalyst CoCuₓO_{y} (the molar ratio of Co:Cu:O is 1:0.9:3.4), 50 ml of sodium hypochlorite solution with a mass concentration of 5.6% were added to a 100 ml round-bottom flask and allowed to react at 30°C for 42 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 121.4% (the calculation methods are the same as those in Example 16).

### Example 37

The preparation of 2,5-furandicarboxylic acid provided in this example was carried out by the following steps: 0.8 g of 5-hydroxymethylfurfural, 1.375 g of the catalyst CoCuₓO_{y} (the molar ratio of Co:Cu:O is 1:0.6:3.0), 50 ml of sodium hypochlorite solution with a mass concentration of 8.27% were added to a 100 ml round-bottom flask and allowed to react at 35°C for 50 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 120.0% (the calculation methods are the same as those in Example 16).

### Example 38

The preparation of 2,5-furandicarboxylic acid provided in this example was carried out by the following steps: in a 100 ml round-bottom flask, 0.9 g of 5-hydroxymethylfurfural was dissolved in 50 ml of aqueous solution, and 1.3 g of the catalyst CoCuₓO_{y} (the molar ratio of Co:Cu:O is 1:0.6:3.0) and ClO₂ in a molar amount 5 times that of 5-hydroxymethylfurfural were added. The mixture was allowed to react at 20°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 99.0% (the calculation methods are the same as those in Example 16).

### Example 39

The preparation of 2,5-furandicarboxylic acid provided in this example was carried out by the following steps: 0.5 g of 5-hydroxymethylfurfural, 0.6 g of co-precipitated catalyst CoCuₓO_{y} (the molar ratio of Co:Cu:O is 1:0.8:3.5), 50 ml of sodium hypochlorite solution with a mass concentration of 4.2% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 70%, and the mass yield of 2,5-furandicarboxylic acid was 71% (the calculation methods are the same as those in Example 16).

### Example 40

The preparation of 2,5-furandicarboxylic acid provided in this example was carried out by the following steps: 0.25 g of 5-hydroxymethylfurfural, 0.125 g of the catalyst CAT-2, 50 ml of sodium hypochlorite solution with a mass concentration of 5.7% were added to a 100 ml round-bottom flask and allowed to react at 5°C for 270 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 119.2% (the calculation methods are the same as those in Example 16).

### Example 41

The preparation of 2,5-furandicarboxylic acid provided in this example was carried out by the following steps: 0.75 g of 5-hydroxymethylfurfural, 0.525 g of the catalyst CAT-2, 50 ml of sodium hypochlorite solution with a mass concentration of 8.3% were added to a 100 ml round-bottom flask and allowed to react at 10°C for 240 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 115.0% (the calculation methods are the same as those in Example 16).

### Example 42

The preparation of 2,5-furandicarboxylic acid provided in this example was carried out by the following steps: 1.0 g of 5-hydroxymethylfurfural, 0.8 g of the catalyst CAT-3, 50 ml of sodium hypochlorite solution with a mass concentration of 10.6% were added to a 100 ml round-bottom flask and allowed to react at 15°C for 200 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 109.7% (the calculation methods are the same as those in Example 16).

### Example 43

The preparation of 2,5-furandicarboxylic acid provided in this example was carried out by the following steps: 1.25 g of 5-hydroxymethylfurfural, 1.125 g of the catalyst CAT-3, 50 ml of sodium hypochlorite solution with a mass concentration of 12.8% were added to a 100 ml round-bottom flask and allowed to react at 17.5°C for 180 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 120.1% (the calculation methods are the same as those in Example 16).

### Example 44

The preparation of 2,5-furandicarboxylic acid provided in this example was carried out by the following steps: 1.5 g of 5-hydroxymethylfurfural, 1.5 g of the catalyst CAT-3, 50 ml of sodium hypochlorite solution with a mass concentration of 14.8% were added to a 100 ml round-bottom flask and allowed to react at 20°C for 150 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 119.0% (the calculation methods are the same as those in Example 16).

### Example 45

The preparation of 2,5-furandicarboxylic acid provided in this example was carried out by the following steps: 1.75 g of 5-hydroxymethylfurfural, 1.93 g of the catalyst CAT-4, 50 ml of sodium hypochlorite solution with a mass concentration of 16.5% were added to a 100 ml round-bottom flask and allowed to react at 22.5°C for 120 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 120.4% (the calculation methods are the same as those in Example 16).

### Example 46

The preparation of 2,5-furandicarboxylic acid provided in this example was carried out by the following steps: 2.0 g of 5-hydroxymethylfurfural, 2.4 g of the catalyst CAT-5, 50 ml of sodium hypochlorite solution with a mass concentration of 18.2% were added to a 100 ml round-bottom flask and allowed to react at 60°C for 10 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 109.6% (the calculation methods are the same as those in Example 16).

### Example 47

The preparation of 2,5-furandicarboxylic acid provided in this example was carried out by the following steps: 2.25 g of 5-hydroxymethylfurfural, 2.8 g of the catalyst CAT-5, 50 ml of sodium hypochlorite solution with a mass concentration of 19.5% were added to a 100 ml round-bottom flask and allowed to react at 57.5°C for 13 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 118.2% (the calculation methods are the same as those in Example 16).

### Example 48

The preparation of 2,5-furandicarboxylic acid provided in this example was carried out by the following steps: 2.5 g of 5-hydroxymethylfurfural, 3.25 g of the catalyst CAT-5, 50 ml of sodium hypochlorite solution with a mass concentration of 20.7% were added to a 100 ml round-bottom flask and allowed to react at 55°C for 15 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 122.4% (the calculation methods are the same as those in Example 16).

### Example 49

The preparation of 2,5-furandicarboxylic acid provided in this example was carried out by the following steps: 2.75 g of 5-hydroxymethylfurfural, 1.35 g of the catalyst CAT-6, 50 ml of sodium hypochlorite solution with a mass concentration of 21.8% were added to a 100 ml round-bottom flask and allowed to react at 52.5°C for 18 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 121.3% (the calculation methods are the same as those in Example 16).

### Example 50

The preparation of 2,5-furandicarboxylic acid provided in this example was carried out by the following steps: 3.0 g of 5-hydroxymethylfurfural, 4.2 g of the catalyst CAT-6, 50 ml of sodium hypochlorite solution with a mass concentration of 22.5% were added to a 100 ml round-bottom flask and allowed to react at 50°C for 20 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 108.6% (the calculation methods are the same as those in Example 16).

### Example 51

The preparation of 2,5-furandicarboxylic acid provided in this example was carried out by the following steps: 3.25 g of 5-hydroxymethylfurfural, 4.7 g of the catalyst CAT-7, 50 ml of sodium hypochlorite solution with a mass concentration of 23.0% were added to a 100 ml round-bottom flask and allowed to react at 47.5°C for 16 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 120.3% (the calculation methods are the same as those in Example 16).

### Example 52

The preparation of 2,5-furandicarboxylic acid provided in this example was carried out by the following steps: 3.5 g of 5-hydroxymethylfurfural, 5.25 g of the catalyst CAT-7, 50 ml of sodium hypochlorite solution with a mass concentration of 23.6% were added to a 100 ml round-bottom flask and allowed to react at 45°C for 14 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 111.0% (the calculation methods are the same as those in Example 16).

### Example 53

The preparation of 2,5-furandicarboxylic acid provided in this example was carried out by the following steps: 3.75 g of 5-hydroxymethylfurfural, 5.8 g of the catalyst CAT-8, 50 ml of sodium hypochlorite solution with a mass concentration of 23.7% were added to a 100 ml round-bottom flask and allowed to react at 40°C for 22 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 115.6% (the calculation methods are the same as those in Example 16).

### Example 54

The preparation of 2,5-furandicarboxylic acid provided in this example was carried out by the following steps: 4.0 g of 5-hydroxymethylfurfural, 6.4 g of the catalyst CAT-8, 50 ml of sodium hypochlorite solution with a mass concentration of 23.6% were added to a 100 ml round-bottom flask and allowed to react at 37.5°C for 25 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 118.3% (the calculation methods are the same as those in Example 16).

### Example 55

The preparation of 2,5-furandicarboxylic acid provided in this example was carried out by the following steps: 4.25 g of 5-hydroxymethylfurfural, 7.0 g of the catalyst CAT-9, 50 ml of sodium hypochlorite solution with a mass concentration of 22.6% were added to a 100 ml round-bottom flask and allowed to react at 35°C for 30 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 105.3% (the calculation methods are the same as those in Example 16).

### Example 56

The preparation of 2,5-furandicarboxylic acid provided in this example was carried out by the following steps: 4.5 g of 5-hydroxymethylfurfural, 7.65 g of the catalyst CAT-9, 50 ml of sodium hypochlorite solution with a mass concentration of 21.25% were added to a 100 ml round-bottom flask and allowed to react at 30°C for 35 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 109.0% (the calculation methods are the same as those in Example 16).

### Example 57

The preparation of 2,5-furandicarboxylic acid provided in this example was carried out by the following steps: 4.75 g of 5-hydroxymethylfurfural, 8.55 g of the catalyst CAT-10, 50 ml of sodium hypochlorite solution with a mass concentration of 19.6% were added to a 100 ml round-bottom flask and allowed to react at 27.5°C for 40 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 104.2% (the calculation methods are the same as those in Example 16).

### Example 58

The preparation of 2,5-furandicarboxylic acid provided in this example was carried out by the following steps: 5.0 g of 5-hydroxymethylfurfural, 10.0 g of the catalyst CAT-10, 50 ml of sodium hypochlorite solution with a mass concentration of 17.7% were added to a 100 ml round-bottom flask and allowed to react at 25°C for 45 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 101% (the calculation methods are the same as those in Example 16).

### Example 59

The preparation of 2,5-furandicarboxylic acid provided in this example was carried out by the following steps: 5.6 g of 5-hydroxymethylfurfural, 1.4 g of the catalyst CAT-2, 50 ml of sodium hypochlorite solution with a mass concentration of 5.6% were added to a 100 ml round-bottom flask and allowed to react at 30°C for 42 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 102.3% (the calculation methods are the same as those in Example 16).

### Example 60

The preparation of 2,5-furandicarboxylic acid provided in this example was carried out by the following steps: 0.8 g of 5-hydroxymethylfurfural, 1.375 g of the catalyst CAT-5, 50 ml of sodium hypochlorite solution with a mass concentration of 8.27% were added to a 100 ml round-bottom flask and allowed to react at 35°C for 50 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 100%, and the mass yield of 2,5-furandicarboxylic acid was 107.0% (the calculation methods are the same as those in Example 16).

### Example 61

The preparation of 2,5-furandicarboxylic acid provided in this example was carried out by the following steps: 0.5 g of 5-hydroxymethylfurfural, 0.6 g of the catalyst CAT-8, 50 ml of sodium hypochlorite solution with a mass concentration of 4.2% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 70%, and the mass yield of 2,5-furandicarboxylic acid was 104.2% (the calculation methods are the same as those in Example 16).

### Comparative Example 1

0.5 g of 5-hydroxymethylfurfural, 0.6 g of the catalyst CoO_{y}, 50 ml of sodium hypochlorite solution with a mass concentration of 4.2% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 63%, and the mass yield of 2,5-furandicarboxylic acid was 31% (the calculation methods are the same as those in Example 1).

### Comparative Example 2

0.5 g of 5-hydroxymethylfurfural, 0.6 g of the catalyst CuO_{y}, 50 ml of sodium hypochlorite solution with a mass concentration of 4.2% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 45%, and the mass yield of 2,5-furandicarboxylic acid was 14% (the calculation methods are the same as those in Example 1).

### Comparative Example 3

0.5 g of 5-hydroxymethylfurfural, 0.49 g of CuCl₂, 50 ml of sodium hypochlorite solution with a mass concentration of 17.7% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 35%, and the mass yield of 2,5-furandicarboxylic acid was 34.6% (the calculation methods are the same as those in Example 1).

### Comparative Example 4

4.25 g of 5-hydroxymethylfurfural, 3.25 g of manganese-cobalt bimetal oxide, 50 ml of sodium hypochlorite solution with a mass concentration of 22.6% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 45.6%, and the mass yield of 2,5-furandicarboxylic acid was 29.7% (the calculation methods are the same as those in Example 1).

### Comparative Example 5

0.8 g of 5-hydroxymethylfurfural, 1.47 g of cerium-titanium composite oxide catalyst, 50 ml of sodium hypochlorite solution with a mass concentration of 21.25% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 58.3%, and the mass yield of 2,5-furandicarboxylic acid was 32.6% (the calculation methods are the same as those in Example 1).

### Comparative Example 6

4.5 g of 5-hydroxymethylfurfural, 3.6 g of the catalyst CoCuₓO_{y} (prepared with calcium perchlorate as the oxidizing agent), 50 ml of sodium hypochlorite solution with a mass concentration of 17.7% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 73%, and the mass yield of 2,5-furandicarboxylic acid was 36.9% (the calculation methods are the same as those in Example 1).

### Comparative Example 7

0.8 g of 5-hydroxymethylfurfural, 0.49 g of the catalyst CoCuₓO_{y} (prepared with N,N-diethylacrylamide as the oxidizing agent), 50 ml of sodium hypochlorite solution with a mass concentration of 17.7% were added to a 100 ml round-bottom flask and allowed to react at 25°C for 60 min at a rotational speed of 400 r/min. At the end of the reaction, the reaction solution was cooled to room temperature to obtain 2,5-furandicarboxylic acid reaction solution. The reaction solution was filtered and then the pH thereof was adjusted to 1-2 by adding an appropriate amount of acid to obtain precipitates, which were centrifuged or filtered to obtain 2,5-furandicarboxylic acid. The raw 2,5-furandicarboxylic acid was finally rinsed with deionized water, suction filtered and vacuum dried to obtain a milky white solid powder of 2,5-furandicarboxylic acid. The conversion rate of 5-hydroxymethylfurfural was 65.3%, and the mass yield of 2,5-furandicarboxylic acid was 40.4% (the calculation methods are the same as those in Example 1).

## Claims

1. A CoCuₓO_{y} catalyst, wherein the molar ratio of Co:Cu:O in the CoCuₓO_{y} catalyst is 1:(0.2-1):(3-4).

2. A method for preparing the CoCuₓO_{y} catalyst according to claim 1, which comprises the following steps:
dispersing a soluble cobalt salt and a soluble copper salt in a mass ratio of 1:(1-2) in water, in which the total mass concentration of these two salts is controlled to be 10% or less;
subsequently, weighing an aqueous solution of a first oxidizing agent and an initiator at a certain mass concentration and adding it dropwise to the mixed aqueous solution of the soluble cobalt salt and the soluble copper salt at a flow rate of 2-5 drops/second;
after the dropwise addition, allowing the mixture to age for 4-6 hours and filtering it to produce a black cake-like precipitate, and roasting the precipitate in an air atmosphere to obtain the CoCuₓO_{y} catalyst.

3. The method of claim 2, wherein the first oxidizing agent is one of or a combination of two or more of perchloric acid, potassium perchlorate, sodium perchlorate, ammonium perchlorate, calcium perchlorate, and magnesium perchlorate.

4. The method of claim 2, wherein the initiator is one of or a combination of two or more of N,N-diethylacrylamide, N,N-dimethylacrylamide, and N-isopropylacrylamide.

5. The method of claim 2, wherein the soluble cobalt salt is one of or a combination of two or more of cobalt chloride, cobalt bromide, cobalt nitrate, and cobalt sulfate.

6. The method of claim 2, wherein the soluble copper salt is one of or a combination of two or more of copper chloride, copper bromide, copper sulfate, and copper nitrate.

7. The method of claim 2, wherein the mass ratio of the first oxidizing agent to the initiator is 1:1.

8. The method of claim 2, wherein the mass concentration of the first oxidizing agent and the initiator is 5-30%.

9. The method of claim 2, wherein the mass concentration of the first oxidizing agent and the initiator is 10-20%.

10. The method of claim 2, which comprises the following steps:
dispersing cobalt nitrate and copper nitrate in a mass ratio of 1:(1-2) in water, in which the total mass concentration of these two salts is controlled to be 10% or less;
subsequently, weighing an aqueous solution of calcium perchlorate and N,N-diethylacrylamide at a certain mass concentration and adding it dropwise to the mixed aqueous solution of copper nitrate and cobalt nitrate at a flow rate of 2-5 drops/second;
after the dropwise addition, allowing the mixture to age for 4-6 hours and filtering it to produce a black cake-like precipitate, and roasting the precipitate in an air atmosphere to obtain the CoCuₓO_{y} catalyst.

11. The method of claim 2 or 10, wherein the roasting is carried out at a temperature of 180°C for 1.5-2.5 hours.

12. The method of claim 10, wherein the mass ratio of calcium perchlorate to N,N-diethylacrylamide is 1:1.

13. The method of claim 10, wherein the mass concentration of calcium perchlorate and N,N-diethylacrylamide is 5-30%.

14. The method of claim 10, wherein the mass concentration of calcium perchlorate and N,N-diethylacrylamide is 10-20%.

15. A method for preparing 2,5-furandicarboxylic acid through catalytic oxidation carried out with the CoCuₓO_{y} catalyst according to claim 1, the method comprises the following steps:
mixing 5-hydroxymethylfurfural, which serves as a raw material, with water, the CoCuₓO_{y} catalyst and a second oxidizing agent in a certain ratio in a reactor, allowing them to react at 20-90°C for 10-600 min, and then cooling to room temperature to obtain 2,5-furandicarboxylic acid reaction solution;
filtering the reaction solution and then adjusting the pH thereof to 1-2 by adding an appropriate amount of an acid to obtain a precipitate, which is centrifuged or filtered, followed by suction filtration and vacuum drying, to obtain 2,5-furandicarboxylic acid.

16. The method according to claim 15, wherein the second oxidizing agent is one of or a combination of two or more of NaClO, KClO and ClO₂.

17. The method according to claim 15, wherein the mass concentration of the 5-hydroxymethylfurfural relative to the total mass of the reaction system is 0.5-25%.

18. The method according to claim 15, wherein the mass concentration of the 5-hydroxymethylfurfural relative to the total mass of the reaction system is 10-20%.

19. The method according to claim 15, wherein the mass ratio of the CoCuₓO_{y} catalyst to 5-hydroxymethylfurfural is 1:1.

20. The method according to claim 15 or 16, wherein the molar ratio of the second oxidizing agent to 5-hydroxymethylfurfural is (1-20): 1.

21. The method according to claim 15 or 16, wherein the molar ratio of the second oxidizing agent to 5-hydroxymethylfurfural is (3-10): 1.

22. The method according to claim 15, wherein the acid used to adjust the pH is one of or a combination of two or more of hydrochloric acid, sulfuric acid, and phosphoric acid.

23. The method according to claim 15, wherein the acid is in a form of an aqueous solution with a mass concentration of 50%.

24. The method according to claim 15, wherein the reaction is carried out at a temperature of 25-35°C for 20-180 minutes.
